(19) Europäisches Patentamt
European Patent Office
Office européen des brevets

(11) **EP 2 974 785 A1**

(12) **EUROPEAN PATENT APPLICATION**

(43) Date of publication:
**20.01.2016 Bulletin 2016/03**

(51) Int Cl.:
**B01D 61/36** (2006.01)     **C07C 29/76** (2006.01)
**C07C 31/04** (2006.01)     **B01J 19/24** (2006.01)

(21) Application number: **14176908.3**

(22) Date of filing: **14.07.2014**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(71) Applicants:
• **Akzo Nobel Chemicals International B.V.**
  **NL-6824 BM Arnhem (NL)**
• **ECN Energieonderzoek Centrum Nederland**
  **1755 LE  Petten (NL)**
• **University Of Twente**
  **7522 NB  Enschede (NL)**

(72) Inventors:
• **Bargeman, Gerrald**
  **NL-6708 NW Wageningen (NL)**

• **van Berkel, Franciscus Petrus Felix**
  **NL-1811 GH Alkmaar (NL)**
• **den Exter, Marcellino Johannes**
  **NL-1816 ME Alkmaar (NL)**
• **de Groot, Matheus Theodorus**
  **NL-3581 RN Utrecht (NL)**
• **Mengers, Harm Jacob**
  **NL-7523 XD Enschede (NL)**
• **Nijmeijer, Dorothea Catharina**
  **NL-7577 SJ Oldenzaal (NL)**
• **van Tuel, Marc Matheus Antonius**
  **NL-1827 RB Alkmaar (NL)**
• **Vos, Hendrik Jan**
  **NL-7313 GK Apeldoorn (NL)**

(74) Representative: **Akzo Nobel IP Department**
**Velperweg 76**
**6824 BM Arnhem (NL)**

(54) **Process for separation methanol and water from non-condensables**

(57)    The present invention pertains to a process for the separation of methanol and water from non-condensables wherein a feed stream comprising methanol, water and non-condensables is subjected to a membrane separation step at a temperature of at least 150°C and a pressure of at least 1 bar (100 kPa) thereby separating the mixture into a permeate being enriched in methanol and water as compared to the feed stream and a retentate being leaner in methanol and water as compared to the feed stream, characterized in that the membrane separation step is carried out using a membrane comprising a separating layer of sulfonated poly ether.

EP 2 974 785 A1

**Description**

*Note*

The work leading to this invention has received funding from the European Union Seventh Framework Programme [FP7/2007- 2013] under grant agreement n° 263007, the CARENA Project.

The present invention relates to a process for the separation of methanol and water from non-condensables using a membrane.

The conventional process for the preparation of methanol is a gas-phase catalytic process where a syngas mixture consisting of CO and $H_2$, and possibly $CO_2$ and $H_2O$, is converted into methanol and water. More particularly, the methanol and water are formed upon heating the syngas mixture to a temperature of between 200 and 250°C and compressing it to a pressure of 50 bar (5 MPa) or higher in the presence of a heterogeneous catalyst. The conversion is limited by thermodynamics to about 20% (depending on syngas composition and conditions). Hence, the produced methanol and water have to be separated from unreacted syngas. This separation is conventionally performed by first cooling down the reactor effluent and subsequently separating condensed methanol and water from the gases in a high pressure gas-liquid separator. The thus obtained gas phase is recycled for re-use in the reactor, while the methanol/water mixture is sent to a distillation column to be separated into the individual components. Relatively high energy consumption due to the repeated cooling, reheating and compression steps is a drawback of this process.

CN102584526 relates to a process for the preparation of methanol from syngas using a membrane reactor. The process comprises the steps of contacting syngas with a catalyst on the reaction side of the membrane reactor at a temperature of between 100 and 300°C and a pressure of between 2 and 10 MPa to convert the syngas into methanol as to form a mixture of the methanol with steam and unreacted syngas, passing the methanol and the steam to the product collection side through the membrane where an absorbent liquid is present so that the methanol and the steam are absorbed and transferred by said absorbent liquid. The syngas is unable to pass through the membrane and is circularly reacted on the reaction side. The absorbent liquid is water, monoethanolamine, propylene glycol dimethyl ether, diethylene glycol dimethyl ether, diethylene glycol butyl ether or a mixture thereof. The membrane materials which can be used are organic materials comprising polyfluortetraethylene, silicon rubber, polyimide; hybrid or composite of organic materials comprising inorganic high molecular materials; or zeolite molecular sieve inorganic materials with a regular hole structure having SAPO-11, SAPO-34, Na-A, K-A, FAU, ZSM-5, Y-molecular sieve and MCM-41 and SBA-15. Energy is saved by performing the separation step at elevated temperature and pressure as energy intensive cooling and reheating and decompressing and compressing steps are avoided. A drawback of this process is however the mandatory use of an absorbent liquid, as the presence of this component in the methanol/ water mixture might adversely affect purification of the methanol or might even require an additional purification step. The regeneration of absorbent liquids required for the re-use of the liquid and the separation from methanol and water normally shows high energy consumption.

It is an object of the present invention to provide a process for separating methanol and water from non-condensables which is energy efficient and wherein an absorbent liquid does not have to be used.

The objective is realized with the process of the present invention wherein methanol and water are separated from non-condensables at elevated temperature using a specific membrane. More particularly, the present invention pertains to a process for the separation of methanol and water from non-condensables wherein a feed stream comprising methanol, water and said non-condensables is subjected to a membrane separation step at a temperature of at least 150°C and a pressure of at least 1 bar (100 kPa) thereby separating the mixture into a permeate being enriched in methanol and water as compared to the feed stream and a retentate being leaner in methanol and water as compared to the feed stream, characterized in that the membrane separation step is carried out using a membrane comprising a separating layer of cross-linked sulfonated poly ether.

The membrane according to the present invention comprises a separating layer of a sulfonated poly ether. The skilled person will be able to prepare such membranes according to or in analogy to methods as described in the art, e.g. by N. Shibuya and R.S. Porter in Macromolecules, 1992, 25, 6495-6499, by C. Bailly et al. in Polymer, 1987, 28, 1009-1016, or by K. Nymeijer et al. in J. of Membrane Science, 2004, 232, 107-114.

In an embodiment of the present invention, the sulfonated poly ether is a crosslinked sulfonated poly ether. Crosslinking can be preferred in order to improve chemical, thermal and/or mechanical resistance of the separating layer. Crosslinking can be achieved via the sulfon groups. However, most preferably, crosslinking is achieved at the main chain of the sulfonated poly ether. Preferably, the sulfonated poly ether is sulfonated poly arylene ether, which can optionally be crosslinked. Even more preferably, the sulfonated poly ether is selected from the group of sulfonated poly arylene ether ketones, and optionally crosslinked. Most preferably, the sulfonated poly ether is sulfonated poly ether ether ketone (SPEEK), which can optionally be crosslinked. SPEEK is commercially available, e.g. by Fumatech GmbH.

Crosslinking of the sulfonated poly ethers, sulfonated poly arylene ether, sulfonated poly arylene ether ketones, and sulfonated poly ether ether ketone may be effectuated using any suitable method as known in the art for the crosslinking of a sulfonated poly ether. Examples of suitable methods can be found in S.D. Mikhailenko et al., Journal of Membrane Science, 2004, Vol. 233(1), 93-99, entitled "Proton conducting membranes based on cross-linked sulfonated poly (ether ether ketone)(SPEEK)"; in P. Knauth et al. in Fuel Cells, 2013, Vol. 13, 79-85, entitled "Proton Mobility in Sulfonated PolyEtherEtherKetone (SPEEK): Influence of Thermal Crosslinking and Annealing"; in H. Li et al. in Journal of Power Sources, 2010, Vol. 195, 8061-8066, entitled "A facile approach to prepare self-cross-linkable sulfonated poly(ether ether ketone) membranes for direct fuel cells"; in G. Merle et al. in Membranes, 2014 4(1), 1-19, entitled "Friedel-Crafts Crosslinked Highly Sulfonated Polyether Ether Ketone (SPEEK) Membranes for a Vanadium/Air Redox Flow Battery"; or in J.-M Song et al. in Macromolecular Research, 2011, Vol. 19(10), 1082-1089, entitled "Preparation and characterization of SPEEK Membranes Crosslinked by Electron Beam Irradiation".

Preferably, the sulfonated poly ether according to the present invention has a crosslinking density of at most 80%. More preferably, it has a crosslinking density of at most 60%, and most preferably it has a crosslinking density of at most 40%. Most preferred is a crosslinking density of between 0 and 40%. As the skilled person will understand, the crosslinking density can be determined via conventional analytical methods, e.g. as described in H. Li et al. in Journal of Power Sources 2010, Vol. 195, 8061-8066, entitled "A facile approach to prepare self-cross-linkable sulfonated poly(ether ether ketone) membranes for direct fuel cells".

The membrane according to the present invention can be supported by a porous support. Preferably, this support is mesoporous (pore diameter between 2 and 50 nm) or microporous (pore diameter < 2 nm). In a preferred embodiment, the support comprises porous alpha alumina. In one embodiment the support comprises macroporous α-alumina with a surface layer composed of γ-alumina. The porous support may also comprise a layered α-alumina structure with decreasing pore sizes to accommodate for the application of a thin γ-layer or other material. The porous support may be made of or comprise porous material selected from any materials known to those skilled in the art including, but not limited to, inorganic porous materials, including, α-alumina, γ-alumina, glass, titania, zirconia, carbon, silicon carbide, clays or silicate materials, aerogels, supported silica, titania, and zirconia; the stainless steels, such as, for example, the 301, 304, 405, 316, 317, and 321 series of stainless steels, the twenty or more HASTELLOY® alloys and the INCONEL® alloys. The membrane of the invention can also be supported by an organic polymer support, which can be a thermoplastic or quasi thermoplastic organic polymer capable of forming porous layers, such as sheets, tubes and the like, and having sufficient heat resistance during the thermal treatment step in manufacturing and having sufficient strength and chemical resistance under the operational conditions. The skilled person will be able to select the appropriate support material on the basis of his general knowledge. Suitable examples include polyacrylonitrile (PAN), polysulfones PSU (including polyphenylsulfones), polyethersulfones (PES), polyether-etherketones (PEEK) and other poly-etherketones, polyimides (PI), including polyether-imide (PEI), polypropylene (PP), polyethylene-terephthalate (PET), polyamides (PA), both aromatic and aliphatic such nylon-6,6, polyamide-imides (PAI), polyvinyldifluoride (PVDF), poly-diorganyl siloxanes, such as polydiphenyl and polydimethyl siloxanes, and cellulose esters. Especially suitable are PAI, PI and PEEK. Also composite materials such as PAN-PA are suitable. Suitable support materials include those in use as ultrafiltration membrane material.

The term "feed stream" as used throughout the application is meant to denote a stream comprising methanol, water and certain non-condensable components. The term "non-condensable components" is meant to denote all components that will not condense at the specified temperature and pressure range, excluding methanol and water. The methanol and water can be present in the feed stream as condensates or as vapours.

The non-condensables in the feed stream which is subjected to the process according to the present invention preferably comprise syngas. Syngas is the name given to a gas mixture that mainly consists of carbon monoxide and hydrogen. Syngas is most commonly produced by steam reforming of natural gas:

$$CH_4 + H_2O \rightarrow CO + 3\ H_2$$

However, the non-condensables in the feed stream can also comprise carbon monoxide, hydrogen, carbon dioxide, a mixture of $CO$, $H_2$ and $CO_2$, a mixture of $CO$ and $CO_2$, or a mixture of $H_2$ and $CO_2$.

Via the process according to the present invention, the feed stream is being separated into a permeate being enriched in methanol and water as compared to the feed stream and a retentate being leaner in methanol and water as compared to the feed stream.

With the wording "a permeate being enriched in methanol and water as compared to the feed stream and a retentate being leaner in methanol and water as compared to the feed stream" is meant that the permeate has a methanol and water selectivity in excess of 1. The methanol and water selectivity S is defined as the ratio of the sum of the mole fractions of methanol and water ($y_{methanol}+y_{water}$) in the permeate over retentate divided by the ratio of the sum of the mole fractions of the non-condensables ($y_{non\text{-}condensables}$) in permeate over retentate. The methanol and water selectivity S is thus defined by:

$$S = \cfrac{\cfrac{\left(y_{methanol} + y_{water}\right)_{permeate}}{\left(y_{methanol} + y_{water}\right)_{retentate}}}{\cfrac{\left(y_{non\text{-}condensables}\right)_{permeate}}{\left(y_{non\text{-}condensables}\right)_{retentate}}}$$

For the sake of clarity, mole fraction of component i ($y_i$) is defined as the number of moles of component i divided by the total number of moles of the components present in the specific phase.

The process of the present invention thus has a methanol and water selectivity S in excess of 1, preferably in excess of 5, more preferably in excess of 10, even more preferably in excess of 50, even more preferably in excess of 100 and most preferably in excess of 1000.

Feed streams which are suitable for being subjected to the separation process according to the present invention include product streams obtained from methanol preparation processes wherein carbon dioxide is hydrogenated. Such a process is for instance known from WO 2007/108014 and WO 2013/144041. In the latter international patent application, a continuous process for the preparation of methanol is described including the followings steps: a gaseous feed comprising hydrogen and carbon dioxide is contacted with a catalyst at a temperature of between 200 and 300°C and a pressure of between 40 (4 MPa) and 200 bar (20 MPa), thereby forming an outlet stream comprising methanol, water, carbon monoxide, carbon dioxide, and hydrogen, and subsequently cooling said outlet stream and subjecting it to a separation step. The step wherein the outlet stream is cooled to separate the condensable components methanol and water from the non-condensable components in the stream is mentioned to be preferably carried out in a feed-effluent heat-exchanger (FEHE) and subsequently in a cooler, or, optionally, in a reboiler of a stripping column or distillation. By replacing the energy intensive cooling step and subsequent separation step according to WO 2013/144041 by the separation step of the present invention, the energy consumption would be significantly lower. In that case, the separation step is performed at elevated temperature using the same pressure as during the methanol preparation step. Thus, in the process according to the present invention, energy consumption is reduced substantially since reheating and recompression are minimized.

The process of the present invention is carried out at a temperature of at least 150°C, more preferably at least 180°C, and most preferably at least 200°C. It is preferably carried out at a temperature of at most 300°C, more preferably at a temperature of at most 275°C, and most preferably at a temperature of at most 250°C.

The process of the present invention is preferably carried out at a pressure of at least 1 bar (100 kPa), more preferably at least 5 bar (0.5 MPa), even more preferably at least 10 bar (1 MPa), more preferably still at least 20 bar (2 MPa), and most preferably at least 40 bar (4 MPa). It is preferably carried out at a pressure of at most 100 bar (10 MPa), more preferably at a pressure of at most 75 bar (7.5 MPa), and most preferably at a pressure of at most 50 bar (5 MPa). In an embodiment according to the present invention, the feed stream is a product stream obtained from a methanol preparation process wherein carbon dioxide was hydrogenated. In that case, the separation process according to the present invention is preferably carried out at approximately the same pressure as was applied in the methanol preparation process. Said methanol production processes are usually carried out at a pressure of at least 10 bar (1 MPa). However, depending on the catalyst that is used, the methanol can also be produced at a pressure of 1 or 2 bar (100 kPa or 200 kPa) (see for example F. Studt et al. in Nature Chemistry, 2014, published online: 2 March 2014 | DOI: 10.1038/NCHEM.1873) or a pressure of between 2 and 10 bar (200 kPa and 1 MPa).

In another embodiment according to the present invention the separation process is carried out simultaneously with the methanol production in a so-called a membrane reactor. As the skilled person will understand, a membrane reactor is a reactor comprising a membrane or a membrane vessel in which a reaction takes place. Examples of suitable membrane reactors are inert membrane reactors wherein catalytic particles are present on the feed side of the membrane, or catalytic membrane reactors wherein catalytic particles are present inside the membrane or at the surface of the membrane. Examples of such membrane reactors are described by Sun and Khang in "A catalytic membrane reactor: Its performance in comparison with other types of reactors", Ind. Eng. Chem. Res. 1990, Vol 29, pp. 232-238, and Mengers et al. in "Multicomponent mass transfer behavior in catalytic membrane reactors", Chem. Eng. Sci. 2014, http://dx.doi.org/10.1016/j.ces.2014.06.010). More particularly, in that embodiment, methanol is prepared in a membrane reactor by hydrogenation of carbon dioxide in the presence of a catalyst, whereby the membrane in said membrane reactor comprises a separating layer of, optionally cross-linked, sulfonated poly ether. The hydrogenation of carbon dioxide is carried out at a temperature of at least 150°C and a pressure of at least 1 bar (100 kPa). The resulting reaction mixture, the resulting comprising methanol, water, catalyst and non-condensables is in situ separated is the feed stream that is being separated in the membrane separation step according to the present invention into a permeate being enriched in methanol and water as compared to the reaction mixture and a retentate being leaner in methanol and water as compared to the feed stream. This embodiment has the additional advantage that a shift in the reaction equilibrium is achieved thereby increasing conversion of the gaseous reactants. This increase leads to less recompression of the unreacted components, thus giving additional energy savings.

Preferably, the permeate obtained using the process according to the present invention is subsequently further purified and/or concentrated, as for most applications of methanol, it is necessary to remove part if not all of the water. The permeate can for instance be subjected to a conventional methanol water distillation step. The permeate can also be stripped, preferably in a stripping column with trays or packing internals or in a flash vessel, and optionally using a partial condenser, as described in detail in WO2013/144041.

The process according to the present invention is further illustrated by the following non-limiting examples.

EXAMPLES

EXAMPLE

A SPEEK on alumina membrane was prepared according to the following procedure. A 1 m long tubular alumina support obtained from Tami (France) with an outer diameter of 14.0 mm, a wall thickness of 2.0 mm, 35% porosity and a mean pore size of 3.4 $\mu$m was taken. On top of this support an $\alpha$-alumina layer with a thickness of 40 $\mu$m, a porosity of 35% and an average pore size of 0.16 $\mu$m was coated. For this coating procedure a suspension of AKP20 alumina powder obtained from Sumito was prepared. This suspension was used for film coating the outer part of the support at a coating speed of 5 mm/s. Subsequently, the coated layer on the support was sintered. The membrane was sintered by increasing the temperature to 1200°C at a temperature increase rate of 100°C per hour. Subsequently the temperature was maintained at 1200°C for 2 hours and subsequently reduced at a rate of 100°C per hour. On top of this $\alpha$-alumina layer a similar $\alpha$-alumina layer of 40 $\mu$m thickness, a porosity of 34% and an average pore size of 0.16 $\mu$m was coated using the same coating and sintering procedures. On top of this second $\alpha$-alumina layer a $\gamma$-alumina layer of 2 $\mu$m thickness, a porosity of 55-60% and an average pore size of 3-5 nm was coated. For this coating procedure a boehmite sol was made through peptizing of alumina-tri-sec-butoxide and subsequently film coating the $\gamma$-alumina layer with this sol at a rate of 5 mm/s. After drying, the coated support was calcined at 600°C during 5 hours after heating the coated support at a rate of 50°C per hour. After the calcination procedure the coated support was cooled down at a rate of 50°C per hour. The sulphonated polyether-ether ketone (SPEEK) layer was coated on top of the $\gamma$-alumina layer. SPEEK was dissolved in NMP at a 5%wt concentration. This solution was used in the coating procedure. The coating speed used was 5 mm/s. After coating the produced membrane was dried during 18 hours at a temperature of 110°C in a nitrogen atmosphere. A heating and cooling rate of 0.5°C per minute was used. A SPEEK layer of 2 $\mu$m was thus obtained. The membrane thus obtained was cut to a membrane length of 72 mm. The membrane had a membrane surface area of 0.00317 m$^2$.

The SPEEK on alumina membrane thus obtained was used during the vapour permeation experiment. A feed containing 19.2 %mole water, 19.2 %mole methanol, 15.4 %mole $CO_2$ and 46.2 %mole $H_2$ was supplied to the 2 $\mu$m SPEEK on alumina vapour permeation membrane. Temperature and feed pressure were set at 200°C and 50 bar, respectively. A feed flow of 300 normal l/h was used (i.e. liters per hour at standard conditions (absolute pressure of 100 kPa and 298 K)). The pressure at the permeate side was 1 bar. Furthermore, a nitrogen sweep gas was supplied to the permeate side at a rate of 300 l/h. The mole fractions at the permeate side were monitored using a Balzers Omnistar 421 on-line mass spectrometer. A heated stainless steel (316L) capillary inlet was used for continuous sampling into the analyzer. For each species, two-point linear calibrations (single point + baseline) were created. Baseline calibration was performed on all relevant masses under pure $N_2$ conditions.

For the gas specific calibrations, accurate gas mixtures of $H_2$ in $N_2$ (0.48 mol%) and $CO_2$ in $N_2$ (0.10 mol%) were prepared in gas cylinders and released in Tedlar bags. These samples were subsequently used for gas specific calibration at atmospheric pressure. A 7.29 mol% $H_2O$ in $N_2$ gas mixture at atmospheric pressure was achieved by slowly bubbling $N_2$ (flow rate 200 mL/min) through demiwater in an Erlenmeyer at a temperature of exactly 40 °C (vapor pressure of $H_2O$ at 40 °C is 7385 Pa which results in 7.29 mol% $H_2O$ in $N_2$ at 1 atm). Similarly, a 1.60 mol% methanol in $N_2$ mixture was created by cooling liquid methanol in an Erlenmeyer to -13 °C (vapor pressure of methanol at -13 °C is 1625.2 Pa which results in 1.60 mol% methanol in $N_2$ at 1 atm). The flux of the individual components and mole fractions of the feed, and retentate and permeate produced are listed in table 1. On the basis of these mole fractions in permeate and feed selectivities are calculated. These are shown in Table 1 as well. On the basis of the results it is clear that permeate enriched in methanol and water is obtained since the selectivity for water and methanol is strongly in excess of 1.

Table 1: Flux of the individual components through the SPEEK on alumina vapour permeation membrane, mole fractions of the feed and the permeate produced and selectivities of the membrane

|  | Water | Methanol | $CO_2$ | $H_2$ | Water + Methanol | $CO_2$ + $H_2$ |
|---|---|---|---|---|---|---|
| mole fraction in feed $y_{i,feed}$ (mol/mol) | 0.192 | 0.192 | 0.154 | 0.462 | 0.384 | 0.616 |
| mole fraction in retentate $y_{i,retentate}$ (mol/mol) | 0.006 | 0.163 | 0.210 | 0.621 | 0.169 | 0.831 |
| mole fraction in permeate $y_{i,permeate}$ (mol/mol) | 0.686 | 0.270 | 0.006 | 0.038 | 0.956 | 0.044 |
| Flux J (mol/m$^2$/h) | 717 | 282 | 6.6 | 39.7 |  |  |
| Water and methanol selectivity S (-) |  |  |  |  | 106 |  |

**Claims**

1.  A process for the separation of methanol and water from non-condensables wherein a feed stream comprising methanol, water and the non-condensables is subjected to a membrane separation step at a temperature of at least 150°C and a pressure of at least 1 bar (100 kPa) thereby separating the mixture into a permeate being enriched in methanol and water as compared to the feed stream and a retentate being leaner in methanol and water as compared to the feed stream, **characterized in that** the membrane separation step is carried out using a membrane comprising a separating layer of sulfonated poly ether.

2.  Process according to claim 1 wherein the sulfonated poly ether is a sulfonated poly arylene ether, a sulfonated poly arylene ether ketone, or a sulfonated poly ether ether ketone.

3.  Process according to claim 1 or 2 wherein the sulfonated poly ether is a crosslinked sulfonated poly ether.

4.  Process according to any one of claims 1-3 wherein the non-condensables comprise carbon monoxide and/or carbon dioxide.

5.  Process according to claim 4 wherein the non-condensables also comprises hydrogen.

6.  Process according to any one of the preceding claims wherein the membrane further comprises a support.

7.  Process according to claim 6 wherein the support is made of or comprises a porous material selected from the group consisting of α-alumina, γ-alumina, glass, titania, zirconia, carbon, silicon carbide, clays or silicate materials, aerogels, supported silica, titania, and zirconia; the support is made of or comprises stainless steels; or the support is made of or comprises a HASTELLOY® alloy or an INCONEL® alloy.

8.  Process according to any one of the preceding claims wherein the sulfonated poly ether is cross-linked sulfonated poly ether ether ketone (SPEEK).

9.  Process according to any one of the preceding claims wherein the feed stream is a product stream of a process for the preparation of methanol by hydrogenation of carbon dioxide.

10. Process according to any one of the preceding claims comprising the subsequent step of subjecting the permeate to a distillation step.

11. Membrane reactor comprising a membrane which comprises a separating layer of, optionally cross-linked, sulfonated poly ether.

12. Membrane reactor according to claim 11 wherein the sulfonated poly ether is selected from the group consisting of a sulfonated poly arylene ether, a sulfonated poly arylene ether ketone, a sulfonated poly ether ether ketone, a crosslinked sulfonated poly arylene ether, a crosslinked sulfonated poly arylene ether ketone, and a crosslinked poly ether ether ketone.

13. Membrane reactor according to claim 11 or 12 wherein the membrane reactor is an inert membrane reactor wherein catalytic particles are present on the feed side of the membrane or a catalytic membrane reactor wherein catalytic particles are present inside the membrane or at the surface of the membrane.

Europäisches
Patentamt

European
Patent Office

Office européen
des brevets

# EUROPEAN SEARCH REPORT

Application Number

EP 14 17 6908

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| Y,D | CN 102 584 526 A (SHANGHAI BI KE CLEAN ENERGY TECHNOLOGY CO LTD) 18 July 2012 (2012-07-18) * abstract * * examples 1-6 * * claims 1-12 * | 1-13 | INV. B01D61/36 C07C29/76 C07C31/04 B01J19/24 |
| Y | STRUIS R P W J ET AL: "A membrane reactor for methanol synthesis", JOURNAL OF MEMBRANE SCIENCE, ELSEVIER, vol. 113, no. 1, May 1996 (1996-05), pages 93-100, XP004041663, ISSN: 0376-7388, DOI: 10.1016/0376-7388(95)00222-7 * abstract * * pages 94-95 * * page 95; figure 1 * * pages 99-100, paragraph "Conclusions" * | 1-13 | |
| Y | CA 2 186 222 A1 (METHANOL CASALE SA [CH]) 19 October 1995 (1995-10-19) * abstract * * pages 4-6 * * claims 1-14 * * figures 1-3 * | 1-13 | TECHNICAL FIELDS SEARCHED (IPC) B01D C07C B01J |
| Y | ES 2 164 544 A1 (UNIV ZARAGOZA [ES]) 16 February 2002 (2002-02-16) * abstract * * columns 3, 4 * * figures 1, 2 * * claims 1-3 * | 1-13 | |

-/--

The present search report has been drawn up for all claims

| Place of search | Date of completion of the search | Examiner |
|---|---|---|
| Munich | 8 January 2015 | Dunet, Guillaume |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

# EUROPEAN SEARCH REPORT

Application Number

EP 14 17 6908

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | GÉRALDINE MERLE ET AL: "Friedel-Crafts Crosslinked Highly Sulfonated Polyether Ether Ketone (SPEEK) Membranes for a Vanadium/Air Redox Flow Battery", MEMBRANES, vol. 4, no. 1, 30 December 2013 (2013-12-30), pages 1-19, XP055142612, DOI: 10.3390/membranes4010001 | 11,12 | |
| A | * abstract *<br>* page 5; figure 1 * | 1-10,13 | |
| X | J. WOOTTHIKANOKKHAN ET AL: "Methanol permeability and properties of DMFC membranes based on sulfonated PEEK/PVDF blends", JOURNAL OF APPLIED POLYMER SCIENCE, vol. 102, no. 6, 15 December 2006 (2006-12-15), pages 5941-5947, XP55142912, ISSN: 0021-8995, DOI: 10.1002/app.25151 | 11,12 | |
| Y | * abstract *<br>* page 5942; figure 1 *<br>* page 5946; table II * | 1-10,13 | TECHNICAL FIELDS SEARCHED (IPC) |
| X | LI L ET AL: "Sulfonated poly(ether ether ketone) membranes for direct methanol fuel cell", JOURNAL OF MEMBRANE SCIENCE, ELSEVIER, vol. 226, no. 1-2, December 2003 (2003-12), pages 159-167, XP004473658, ISSN: 0376-7388, DOI: 10.1016/J.MEMSCI.2003.08.018 | 11,12 | |
| Y | * abstract *<br>* page 161, paragraph 2.6 *<br>* page 164; table 1 *<br>* page 165; figure 6 * | 1-10,13 | |

-/--

The present search report has been drawn up for all claims

| Place of search | Date of completion of the search | Examiner |
|---|---|---|
| Munich | 8 January 2015 | Dunet, Guillaume |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 14 17 6908

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | V. SILVA ET AL: "Zirconium Oxide Modified Sulfonated Poly (Ether Ether Ketone) Membranes for Direct Methanol Fuel Cell Applications", MATERIALS SCIENCE FORUM, vol. 455-456, January 2004 (2004-01), pages 587-591, XP55142840, DOI: 10.4028/www.scientific.net/MSF.455-456.587 | 11,12 | |
| Y | * abstract * <br> * figure 5 * <br> * page 591 * <br> ----- | 1-10,13 | |

TECHNICAL FIELDS SEARCHED (IPC)

The present search report has been drawn up for all claims

| Place of search | Date of completion of the search | Examiner |
|---|---|---|
| Munich | 8 January 2015 | Dunet, Guillaume |

EPO FORM 1503 03.82 (P04C01)

## ANNEX TO THE EUROPEAN SEARCH REPORT
## ON EUROPEAN PATENT APPLICATION NO.

EP 14 17 6908

This annex lists the patent family members relating to the patent documents cited in the above-mentioned European search report.
The members are as contained in the European Patent Office EDP file on
The European Patent Office is in no way liable for these particulars which are merely given for the purpose of information.

08-01-2015

| Patent document cited in search report | | Publication date | Patent family member(s) | | Publication date |
|---|---|---|---|---|---|
| CN 102584526 | A | 18-07-2012 | NONE | | |
| CA 2186222 | A1 | 19-10-1995 | AU | 2065595 A | 30-10-1995 |
| | | | CA | 2186222 A1 | 19-10-1995 |
| | | | CH | 687004 A5 | 30-08-1996 |
| | | | CN | 1147241 A | 09-04-1997 |
| | | | EP | 0754172 A1 | 22-01-1997 |
| | | | JP | H09511509 A | 18-11-1997 |
| | | | WO | 9527690 A1 | 19-10-1995 |
| ES 2164544 | A1 | 16-02-2002 | NONE | | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- CN 102584526
- WO 2007108014 A
- WO 2013144041 A

### Non-patent literature cited in the description

- **N. SHIBUYA ; R.S. PORTER.** *Macromolecules,* 1992, vol. 25, 6495-6499
- **C. BAILLY et al.** *Polymer,* 1987, vol. 28, 1009-1016
- **K. NYMEIJER et al.** *J. of Membrane Science,* 2004, vol. 232, 107-114
- **S.D. MIKHAILENKO et al.** Proton conducting membranes based on cross-linked sulfonated poly (ether ether ketone)(SPEEK). *Journal of Membrane Science,* 2004, vol. 233 (1), 93-99
- **P. KNAUTH et al.** Proton Mobility in Sulfonated PolyEtherEtherKetone (SPEEK): Influence of Thermal Crosslinking and Annealing. *Fuel Cells,* 2013, vol. 13, 79-85
- **H. LI et al.** A facile approach to prepare self-cross-linkable sulfonated poly(ether ether ketone) membranes for direct fuel cells. *Journal of Power Sources,* 2010, vol. 195, 8061-8066
- **G. MERLE et al.** Friedel-Crafts Crosslinked Highly Sulfonated Polyether Ether Ketone (SPEEK) Membranes for a Vanadium/Air Redox Flow Battery. *Membranes,* 2014, vol. 4 (1), 1-19
- **J.-M SONG et al.** Preparation and characterization of SPEEK Membranes Crosslinked by Electron Beam Irradiation. *Macromolecular Research,* 2011, vol. 19 (10), 1082-1089
- **F. STUDT et al.** *Nature Chemistry,* 02 March 2014
- **SUN ; KHANG.** A catalytic membrane reactor: Its performance in comparison with other types of reactors. *Ind. Eng. Chem. Res.,* 1990, vol. 29, 232-238
- **MENGERS et al.** Multicomponent mass transfer behavior in catalytic membrane reactors. *Chem. Eng. Sci,* 2014, http://dx.doi.org/10.1016/j.ces.2014.06.010